# EUROPEAN PATENT APPLICATION

(11) **EP 4 574 825 A1**
(43) Date of publication of application: **25.06.2025**
(21) Application number: 23854299.7
(22) Date of filing: 09.08.2023
(51) Int. Cl.: C07D 487/04, A61P 35/00, A61P 35/02

(54) **CRYSTAL FORM OF PYRAZOLOPYRIMIDINE ESTER COMPOUND AND PREPARATION METHOD THEREFOR**

(30) Priority: 17.08.2022 CN 202210984527
(71) Applicant: Shanghai Maius Pharmaceutical Co. Ltd., Shanghai 201210 (CN)
(72) Inventor: GUO, Yekun, Shanghai 201210 (CN); HU, Xiang, Shanghai 201210 (CN); HUANG, Dujian, Shanghai 201210 (CN); SHI, Mingfeng, Shanghai 201210 (CN)
(74) Representative: Plasseraud IP
(86) International application number: PCT/CN2023/111918
(87) International publication number: WO 2024/037396

(57) **Abstract**

The present invention provides a crystalline form Form II of a pyrazolopyrimidine ester compound of the structure of formula (I)

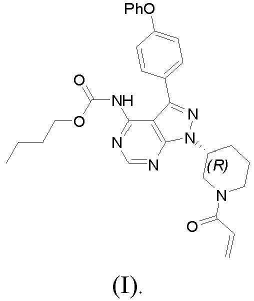

This crystalline form has been characterized using X-ray powder diffraction (XRPD), differential scanning calorimetry (DSC) and infrared (IR) spectroscopy. The present invention also provides a method of preparing the crystalline form Form II of the pyrazolopyrimidine ester compound. This crystalline form allows long-term storage without special requirements in respect of temperature, light, humidity or oxygen level and is significantly advantageous in terms of stability. Moreover, the method involves simple steps and provides good reproducibility and excellent purity. It has a promising prospect of extensive application.

## Description

### TECHNICAL FIELD

The present invention relates to the field of chemical medicines and, in particular, to a crystalline form of a pyrazolopyrimidine ester compound and a method of preparing the same.

### BACKGROUND

Bruton's tyrosine kinase (BTK) is a non-receptor cytoplasmic tyrosine protein kinase of the Tec family. BTK is involved in the transduction of TLR, BAFF-R, BCR and CXCR4/5 signals, as well as in the proliferation, differentiation, apoptosis and migration of regulatory B-cells. As BTK plays a crucial role in pathogenesis of malignant B-cell lymphoma, BTK inhibitors are used as important drugs for B-cell lymphoma treatment.

CN 202111131059.0 discloses a series of pyrazolopyrimidine ester compounds as BTK inhibitor prodrugs, which, once orally administered, will be rapidly absorbed into the blood and hydrolyzed into the active metabolite 1-[(3R)-3-[4-amino-3-(4-phenoxyphenyl)-1H-pyrazolo[3,4-d]pyrimidin-1-yl]-1-piperidinyl ]-2-propen-1-one. This is known as the 4-amino active metabolite (4-AAM), an active metabolite commonly recognized in current clinical practice to be able to exert the intended therapeutic effect. Among these compounds, the one chemically named as 1-[(3R)-3-[4-(butoxycarbonyl)-amino-3-(4-phenoxyphenyl)-1H-pyrazolo[3,4-d]pyrimidin-1-yl]-1-piperidinyl]-2-propen-1-one and of the chemical structure represented by formula (I) possess the most desirable properties as a prodrug because it is hydrolyzed most rapidly in blood and has the highest 4-AAM AUC while itself showing the lowest AUC.

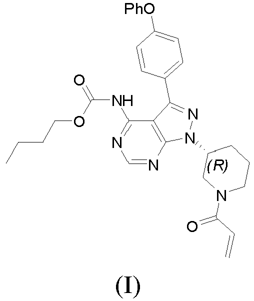

CN 202111131059.0 also provides a method for synthesis of this compound. A product obtained according to this method was found to be amorphous. The compound of formula (I) can be obtained by reacting commercially available 1-[(3R)-3-[4-amino-3-(4-phenoxyphenyl)-1H-pyrazolo[3,4-d]pyrimidin-1-yl]-1-piperidyl]-2-propen-1-one as one of the reactants with butyl chloroformate. Studies have shown that this amorphous compound exhibits poor stability and is susceptible to heat and moisture. Significant degradation can be observed after its storage over 10 days at 40 °C, and almost complete degradation can be observed after storage over 10 days at 60 °C and 75% RH. Consequently, undesirable substances may emerge under regular storage conditions, creating great difficulties in the storage and processing of the product. Considering the potential clinical value of compounds of formula (I), it is particularly important to obtain its crystalline form with desirable purity and stable properties.

### SUMMARY

A stable and reproducible crystalline form is developed by the applicant and named as Form II. Also provided herein is a method of preparing Form II. The crystalline form Form II is significantly advantageous in terms of stability by allowing long-term storage without special requirements in respect of temperature, light, humidity or oxygen level.

It is a first object of the present invention to provide a crystalline form of a pyrazolopyrimidine ester compound. To this end, the present invention provides the technical solution as below:

A crystalline form Form II of a pyrazolopyrimidine ester compound of the structure of formula (I), which has three or more (preferably four or more, five or more, six or more, seven or more, or eight) characteristic peaks expressed in degrees 2θ at 5.53°, 9.82°, 10.76°, 17.87°, 20.55°, 21.28°, 21.92° and 25.15° in its X-ray powder diffraction (XRPD) pattern, wherein the error range of degrees 2θ is ± 0.20°, and the XRPD pattern is obtained using Cu-Kα radiation.

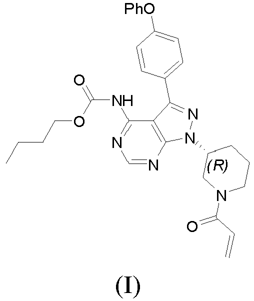

Additionally, the crystalline form Form II has characteristic peaks expressed in degrees 2θ at 5.53°, 9.82°, 10.76°, 14.25°, 14.81°, 16.72°, 17.87°, 18.33°, 19.06°, 19.67°, 20.55°, 20.78°, 21.28°, 21.92°, 22.93°, 23.38°, 23.85°, 25.15°, 26.09°, 26.51°, 26.97°, 27.52°, 27.90°, 28.37°, 29.00°, 29.70°, 31.57°, 32.57°, 33.94° and 36.71° in its XRPD pattern, wherein the error range of degrees 2θ is ± 0.20°.

Additionally, the degrees 2θ and relative intensities I (expressed as percentages to the most intense diffraction peak) of the crystalline form Form II in its XRPD pattern are as listed in the table below:

| 2θ ± 0.2(°) | Relative Intensity I | 2θ ± 0.2(°) | Relative Intensity I |
|---|---|---|---|
| 5.53° | 56.60% | 23.38° | 5.00% |
| 9.82° | 40.00% | 23.85° | 10.20% |
| 10.76° | 46.60% | 25.15° | 25.80% |
| 14.25° | 21.90% | 26.09° | 3.10% |
| 14.81° | 16.20% | 26.51° | 4.20% |
| 16.72° | 13.60% | 26.97° | 4.30% |
| 17.87° | 100.00% | 27.52° | 2.60% |
| 18.33° | 4.60% | 27.90° | 6.40% |
| 19.06° | 20.50% | 28.37° | 8.10% |
| 19.67° | 18.90% | 29.00° | 3.90% |
| 20.55° | 26.60% | 29.70° | 3.40% |
| 20.78° | 16.40% | 31.57° | 2.90% |
| 21.28° | 26.00% | 32.57° | 2.10% |
| 21.92° | 57.60% | 33.94° | 4.00% |
| 22.93° | 9.00% | 36.71° | 2.60% |

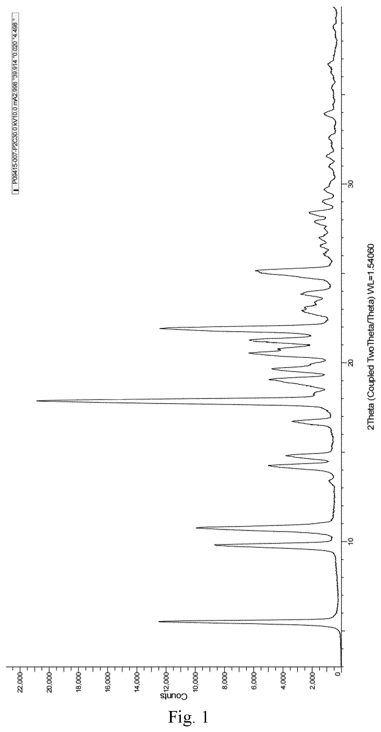

Additionally, the crystalline form Form II is characterized by the XRPD pattern as shown in Fig. 1.

Additionally, the crystalline form Form II is an irregularly-shaped crystalline form.

Additionally, the crystalline form Form II has an endothermic peak at 129.59 °C in its differential scanning calorimetry (DSC) curve, wherein the error range of the endothermic peak temperature is ± 1.00 °C.

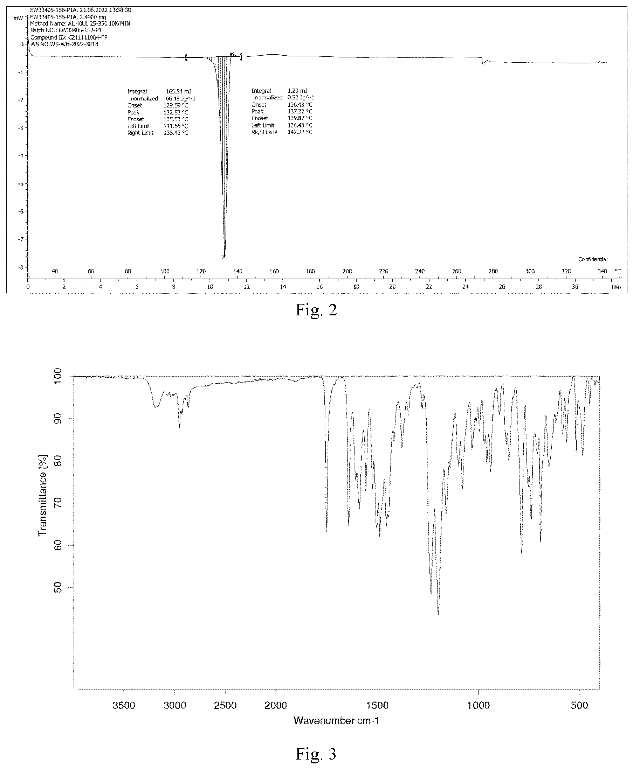

Additionally, the crystalline form Form II is characterized by the DSC curve as shown in Fig. 2.

Additionally, the crystalline form Form II shows characteristic absorption bands at the wavenumbers listed in the table below in its infrared (IR) spectrum, wherein the error range of the absorption band peaks is ± 2 cm⁻¹; "w" (weak), "m" (medium) or "s" (strong) added to indicate the intensity of the peak.

| Wavenumber (cm⁻¹) | Wavenumber (cm⁻¹) | Wavenumber (cm⁻¹) | Wavenumber (cm⁻¹) |
|---|---|---|---|
| 3195w | 2928w | 1556m | 1233s |
| 3166w | 2897w | 1504m | 1197s |
| 3076w | 2865w | 1488s | 1138m |
| 3044w | 1750m | 1455m | 1078m |
| 3027w | 1642m | 1445m | 1030w |
| 3012w | 1606m | 1376w | |
| 2954w | 1588m | 1277w | |

Additionally, the crystalline form Form II shows characteristic absorption bands at wavenumbers of 423, 449, 485, 517, 564, 584, 616, 652, 679, 693, 708, 739, 754, 787, 827, 849, 862, 896, 941, 959, 971, 996, 1011, 1030, 1078, 1095, 1138, 1159, 1197, 1233, 1277, 1303, 1345, 1376, 1416, 1445, 1455, 1488, 1504, 1524, 1556, 1588, 1606, 1642, 1711, 1750, 1901, 2865, 2897, 2928, 2954, 3012, 3027, 3044, 3076, 3166 and 3195 cm⁻¹ in IR spectrum, wherein the error range of the absorption band peaks is 2 cm⁻¹.

Additionally, the crystalline form Form II is characterized by the IR spectrum as shown in Fig. 3.

Additionally, the crystalline form Form I is almost non-hygroscopic as determined by dynamic vapor sorption (DVS). The XRPD pattern of the crystalline form Form II remained the same before and after the DVS determination.

Additionally, the crystalline form Form II showed a lower degree of degradation than the amorphous form under several forced degradation conditions in a stability study. In particular, the crystalline form Form I samples were very stable over 10 days of storage at room temperature and in light. The crystalline form Form I can be stored under regular conditions without special protection as the study suggests, which is particularly important in pharmaceutical production.

It is a second object of the present invention to provide a method of preparing the crystalline form Form II of the pyrazolopyrimidine ester compound as set forth above by crystallization of the compound of formula (I) in the amorphous or crystalline (Form I) form.

To prepare the crystalline form Form II by means of crystallization of the compound of formula (I) in the amorphous form, high purity of the amorphous compound of formula (I) is required to be not lower than 98.5%; or, by means of addition of crystal seeds in the crystalline form Form II for inducing the formation of the crystalline form Form II, high purity of the amorphous compound of formula (I) is not required.

In the preparation of crystalline form Form II from crystalline form Form I of the compound of formula (I), the crystalline form Form I is prepared by crystallizing the amorphous compound of formula (I). The crystalline form Form I has three or more (preferably four or more, five or more, six or more, seven or more, or eight) characteristic peaks expressed in degrees 2θ at 11.26°, 14.03°, 14.80°, 17.07°, 19.78°, 21.21°, 22.39° and 23.85° in its XRPD pattern, wherein the error range of degrees 2θ is ± 0.20°, and the XRPD pattern is obtained using Cu-Kα radiation.

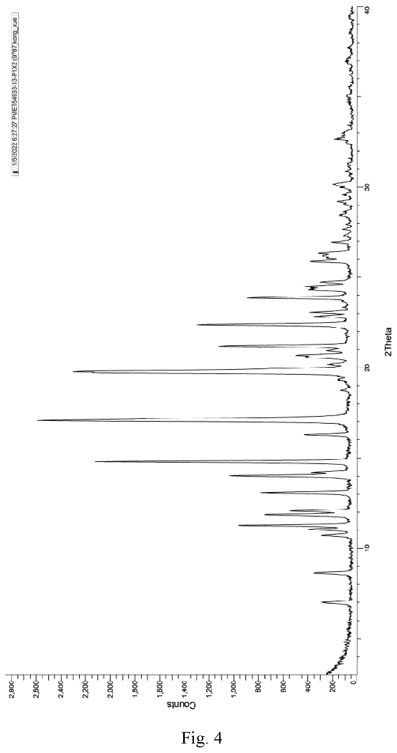

Additionally, the crystalline form Form I has characteristic peaks expressed in degrees 2θ at 6.99°, 8.62°, 10.72°, 11.05°, 11.26°, 11.86°, 12.07°, 13.08°, 14.03°, 14.80°, 16.28°, 17.07°, 19.33°, 19.78°, 20.19°, 20.69°, 21.21°, 22.39°, 22.86°, 23.08°, 23.85°, 24.40°, 24.73°, 25.95°, 26.23°, 26.95°, 29.21°, 30.17°, 32.71° and 32.99° in its XRPD pattern, wherein the error range of degrees 2θ is ± 0.20°. The crystalline form Form I is characterized by the XRPD pattern as shown in Fig. 4.

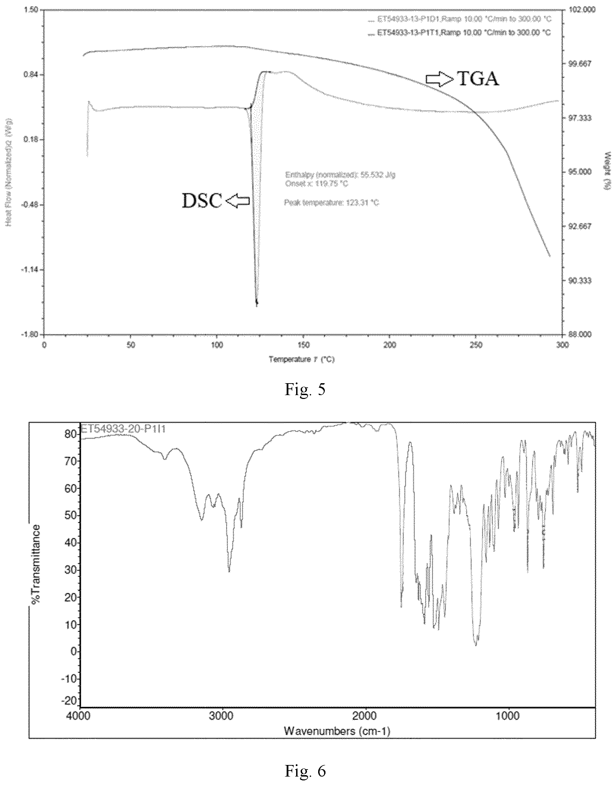

Additionally, the crystalline form Form I is an irregularly-shaped crystalline form. The crystalline form Form I has an endothermic peak at 119.75 °C in its DSC curve, wherein the error range of the endothermic peak temperature is ± 1.00 °C. In thermogravimetric analysis (TGA), the crystalline form Form I exhibits no significant weight loss before degradation, indicating that Form I is an anhydrous crystalline form. The crystalline form Form I is characterized by the DSC and TGA curves as shown in Fig. 5.

Additionally, the crystalline form Form I has characteristic absorption bands at wavenumbers of 493, 519, 587, 609, 676, 692, 759, 774, 794, 808, 869, 895, 934, 963, 1000, 1027, 1073, 1102, 1134, 1158, 1229, 1342, 1382, 1448, 1490, 1523, 1559, 1589, 1645, 1681, 1753, 2870, 2954, 3067, 3149 and 3406 cm⁻¹ in its IR spectrum, wherein the error range of the absorption band peaks is ± 2 cm⁻¹. The crystalline form Form I is characterized by the IR spectrum as shown in Fig. 6.

The crystalline form Form I, from which the crystalline form Form II can be prepared, may be prepared by crystallization according to the method comprising the steps of: adding the amorphous compound of formula (I) to a mixed solvent; and stirring the resulting suspension, followed by filtering at room temperature and drying, affording the pyrazolopyrimidine ester compound in the crystalline form Form I, wherein the mixed solvent is obtained by mixing a good solvent with a poor solvent; wherein the good solvent is a solvent, in which the compound of formula (I) is soluble, and is selected from methanol, ethanol, isopropanol, acetonitrile, acetone, butanone, ethyl acetate, isopropyl acetate and tetrahydrofuran; the poor solvent is a solvent, in which the compound of formula (I) is insoluble, sparingly soluble or slightly soluble, and is selected from methyl tert-butyl ether, water, n-heptane and cyclohexane; the mixed solvent is used at a volume-to-weight (v/w) ratio of 5 to 150 to the compound of formula (I), and in the mixed solvent, the poor solvent and the good solvent are used at a volume-to-volume (v/v) ratio of (1-14) : 1, where v is measured in milliliters (mL), and w is measured in grams (g).

Specifically, the method of preparing the crystalline form Form II from the amorphous or crystalline (Form I) compound of formula (I) comprising the steps of:
dissolving the pyrazolopyrimidine ester compound of the structure of formula (I) in a good solvent; then adding a poor solvent (anti-solvent) to precipitate a solid; and stirring, followed by filtering and drying, affording the pyrazolopyrimidine ester compound in the crystalline form Form II; wherein the good solvent is a solvent, in which the compound of formula (I) is soluble, and the poor solvent is a solvent, in which the compound of formula (I) is insoluble, sparingly soluble or slightly soluble.

Additionally, the poor solvent is added slowly in batches, preferably in two batches.

Additionally, the good solvent is selected from acetone, 2-methyltetrahydrofuran, methanol, acetonitrile, ethyl acetate and tetrahydrofuran, with acetone or 2-methyltetrahydrofuran being preferred, and the poor solvent is selected from *n*-heptane, methyl *tert*-butyl ether, water and cyclohexane, with *n*-heptane being preferred.

Additionally, in the method of preparing the crystalline form Form II, the good solvent is used at a volume-to-weight (v/w) ratio of 5 to 10 to the compound of formula (I), and the poor solvent is used at a volume-to-weight (v/w) ratio of 10 to 150 to the compound of formula (I), wherein v is measured in milliliters (mL), and w is measured in grams (g).

Additionally, in the method of preparing the crystalline form Form II, the good solvent is used at a volume-to-weight (v/w) ratio of 5-7 to the compound of formula (I), and the poor solvent is used at a volume-to-weight (v/w) ratio of 14-21 to the compound of formula (I).

It is a third object of the present invention to provide use of the crystalline form Form II of the pyrazolopyrimidine ester compound as set forth above for the preparation of medicaments for treating blood diseases such as lymphoma and lymphocytic leukemia.

The present invention offers the following benefits:
The pyrazolopyrimidine ester compound in the crystalline form Form II can be obtained according to the method set forth in the present invention. This crystalline form allows long-term storage without special requirements in respect of temperature, light, humidity or oxygen level and is significantly superior over both the amorphous form and the crystalline form Form I. In addition, the method involves simple steps and provides good reproducibility and excellent purity. Therefore, it has a promising prospect of extensive application.

### BRIEF DESCRIPTION OF THE DRAWINGS

Fig. 1 shows an X-ray powder diffraction (XRPD) spectrum of a crystalline form Form II of a pyrazolopyrimidine ester compound.
Fig. 2 shows a differential scanning calorimetry (DSC) curve of the crystalline form Form II of the pyrazolopyrimidine ester compound.
Fig. 3 shows an infrared (IR) spectrum of the crystalline form Form II of the pyrazolopyrimidine ester compound.
Fig. 4 shows an XRPD pattern of a crystalline form Form I of the pyrazolopyrimidine ester compound.
Fig. 5 shows DSC and thermogravimetric analysis (TGA) curves of the crystalline form Form I of the pyrazolopyrimidine ester compound.
Fig. 6 shows an IR spectrum of the crystalline form Form I of the pyrazolopyrimidine ester compound.

### DETAILED DESCRIPTION

The present invention will be described clearly and fully below with respect to specific embodiments thereof. It should be understood that the embodiments set forth herein are merely some, but not all, of the possible embodiments of this invention. Any and all other embodiments devisable by skilled artisans in light of the disclosed embodiments without paying any creative effort are considered to fall within the scope of the invention.

In the context of the present invention, room temperature is defined as a temperature in the range of 20 to 30 °C, preferably 25 °C.

In the following examples, the ratios of the good and poor solvents used to the compound of formula (I) are all enclosed in round brackets. For example, "(5V)" denotes that the solvent is used at a ratio (v/w) of 5 to the compound of formula (I), where v is measured in milliliters (mL) and w in grams (g).

The amorphous compound of formula (I) used in the following examples can be prepared according to the method disclosed in CN202111131059.0: the reactants 1-[(3R)-3-[4-amino-3-(4-phenoxyphenyl)-1H-pyrazolo[3,4-d]pyrimidin-1-yl]-1-piperidyl]-2-propen-1-one, butyl chloroformate and dichloromethane were added under nitrogen protection; the mixture was cooled to 10 °C; pyridine was added dropwise and the reaction mixture was stirred for 3-4 hours at this temperature; ice water was added to quench the reaction when TLC shows that almost no reactants remain; aqueous phase was separated and extracted with dichloromethane; organic phases were combined and washed with water. The organic phase was concentrated and the residue was purified by column chromatography to afford the compound of formula (I).

The compound of formula (I) in the crystalline form Form I used in the following examples can be prepared according to the method set forth above. Specifically, the method may comprises the steps of: weighing a certain amount of the amorphous compound of the formula (I) and adding it to a mixed solvent (10V), wherein the mixed solvent is obtained by mixing methyl tert-butyl ether as a poor solvent with ethanol as a good solvent at a volume-to-volume ratio of 9: 1; stirring the resulting suspension at room temperature for 3 days and then filtering it; and drying the resulting solid, affording the pyrazolopyrimidine ester compound in the crystalline form Form I.

### Example 1

200 g of the amorphous compound of the formula (I) with purity of 98.5% was weighed and dissolved at room temperature (20-30 °C) in 1000 mL (5V) of acetone as a good solvent. 600 mL (3V) of *n*-heptane was then added as a poor solvent to slowly precipitate a solid. Stirring was carried out at 5-15 °C for 2 h, and 2400 mL (12V) of *n*-heptane was then slowly added dropwise within 3 h. After the addition was completed, stirring was continued at 0-10 °C for 2 h, followed by filtering. The resulting solid was dried under vacuum at 50 °C for 4.5 h, giving a solid at a purification yield of 82.5%. The resulting solid was subjected to XRPD and DSC analysis, and the results showed that the pyrazolopyrimidine ester compound was obtained in the crystalline form Form II.

### Example 2

200 g of the amorphous compound of the formula (I) with purity of 96% was weighed and dissolved at room temperature (20-30 °C) in 1000 mL (5V) of acetone as a good solvent. 600 mL (3V) of *n*-heptane as a poor solvent and 0.2 g of crystal seeds in crystalline form Form II were then added to slowly precipitate a solid. Stirring was carried out at 5-15 °C for 2 h, and 2400 mL (12V) of *n*-heptane was then slowly added dropwise within 3 h. After the addition was completed, stirring was continued at 0-10 °C for 2 h, followed by filtering. The resulting solid was dried under vacuum at 50 °C for 4.5 h, giving a solid at a purification yield of 80.5%. The resulting solid was subjected to XRPD and DSC analysis, and the results showed that the pyrazolopyrimidine ester compound was obtained in the crystalline form Form II.

### Example 3

200 g of the compound of the formula (I) in the crystalline form Form I was weighed and dissolved at room temperature (20-30 °C) in 1000 mL (5V) of acetone as a good solvent. 600 mL (3V) of *n*-heptane was then added as a poor solvent to slowly precipitate a solid. Stirring was carried out at 5-15 °C for 2 h, and 2400 mL (12V) of *n*-heptane was then slowly added dropwise within 3 h. After the addition was completed, stirring was continued at 0-10 °C for 2 h, followed by filtering. The resulting solid was dried under vacuum at 50 °C for 4.5 h, giving a solid at a purification yield of 81.1%. The resulting solid was subjected to XRPD and DSC analysis, and the results showed that the pyrazolopyrimidine ester compound was obtained in the crystalline form Form II.

### Example 4

30 g of the amorphous compound of the formula (I) with purity of 98.5% was weighed and dissolved at room temperature (20-30 °C) in 150 mL (5V) of acetone as a good solvent. 90 mL (3V) of n-heptane was then added as a poor solvent to slowly precipitate a solid. Stirring was carried out at 5-15 °C for 2 h, and 360 mL (12V) of n-heptane was then slowly added dropwise within 2.5 h. After the addition was completed, stirring was continued at 0-10 °C for 3 h, followed by filtering. The resulting solid was dried under vacuum at 50 °C for 4.5 h, giving a solid at a purification yield of 84.1%. The resulting solid was subjected to XRPD and DSC analysis, and the results showed that the pyrazolopyrimidine ester compound was obtained in the crystalline form Form II.

### Example 5

30 g of the amorphous compound of the formula (I) with purity of 96% was weighed and dissolved at room temperature (20-30 °C) in 150 mL (5V) of acetone as a good solvent. 90 mL (3V) of *n*-heptane as a poor solvent and 0.03 g of crystal seeds in the crystalline form Form II were then added to slowly precipitate a solid. Stirring was carried out at 5-15 °C for 2 h, and 360 mL (12V) of *n*-heptane was then slowly added dropwise within 2.5 h. After the addition was completed, stirring was continued at 0-10 °C for 3 h, followed by filtering. The resulting solid was dried under vacuum at 50 °C for 4.5 h, giving a solid at a purification yield of 81.0%. The resulting solid was subjected to XRPD and DSC analysis, and the results showed that the pyrazolopyrimidine ester compound was obtained in the crystalline form Form II.

### Example 6

30 g of the compound of the formula (I) in the crystalline form Form I was weighed and dissolved at room temperature (20-30 °C) in 150 mL (5V) of acetone as a good solvent. 90 mL (3V) of *n*-heptane was then added as a poor solvent to slowly precipitate a solid. Stirring was carried out at 5-15 °C for 2 h, and 360 mL (12V) of *n*-heptane was then slowly added dropwise within 2.5 h. After the addition was completed, stirring was continued at 0-10 °C for 3 h, followed by filtering. The resulting solid was dried under vacuum at 50 °C for 4.5 h, giving a solid at a purification yield of 84.5%. The resulting solid was subjected to XRPD and DSC analysis, and the results showed that the pyrazolopyrimidine ester compound was obtained in the crystalline form Form II.

### Example 7

5 g of the amorphous compound of the formula (I) with purity of 98.5% was weighed and dissolved at 5-15 °C in 35 mL (7V) of acetone as a good solvent. 25 mL (5V) of *n*-heptane was then added as a poor solvent to slowly precipitate a solid. Stirring was carried out at 5-15 °C for 1 h, and 45 mL (9V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 41.0%. The resulting solid was subjected to XRPD and DSC analysis, and the results showed that the pyrazolopyrimidine ester compound was obtained in the crystalline form Form II.

### Example 8

5 g of the amorphous compound of the formula (I) with purity of 96% was weighed and dissolved at 5-15 °C in 35 mL (7V) of acetone as a good solvent. 25 mL (5V) of *n*-heptane as a poor solvent and 0.005 g of crystal seeds in the crystalline form Form II were then added to slowly precipitate a solid. Stirring was carried out at 5-15 °C for 1 h, and 45 mL (9V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 38.9%. The resulting solid was subjected to XRPD and DSC analysis, and the results showed that the pyrazolopyrimidine ester compound was obtained in the crystalline form Form II.

### Example 9

5 g of the compound of the formula (I) in the crystalline form Form I was weighed and dissolved at 5-15 °C in 35 mL (7V) of acetone as a good solvent. 25 mL (5V) of *n*-heptane was then added as a poor solvent to slowly precipitate a solid. Stirring was carried out at 5-15 °C for 1 h, and 45 mL (9V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 42.7%. The resulting solid was subjected to XRPD and DSC analysis, and the results showed that the pyrazolopyrimidine ester compound was obtained in the crystalline form Form II.

### Example 10

5 g of the amorphous compound of the formula (I) with purity of 98.5% was weighed and dissolved at 5-15 °C in 35 mL (7V) of acetone as a good solvent. 25 mL (5V) of n-heptane was then added as a poor solvent to slowly precipitate a solid. Stirring was carried out at 5-15 °C for 1 h, and 80 mL (16V) of n-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 48.0%. The resulting solid was subjected to XRPD and DSC analysis, and the results showed that the pyrazolopyrimidine ester compound was obtained in the crystalline form Form II.

### Example 11

5 g of the amorphous compound of the formula (I) with purity of 96% was weighed and dissolved at 5-15 °C in 35 mL (7V) of acetone as a good solvent. 25 mL (5V) of n-heptane as a poor solvent and 0.005 g of crystal seeds in the crystalline form Form II were then added to slowly precipitate a solid. Stirring was carried out at 5-15 °C for 1 h, and 80 mL (16V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 47.2%. The resulting solid was subjected to XRPD and DSC analysis, and the results showed that the pyrazolopyrimidine ester compound was obtained in the crystalline form Form II.

### Example 12

5 g of the compound of the formula (I) in the crystalline form Form I was weighed and dissolved at 5-15 °C in 35 mL (7V) of acetone as a good solvent. 25 mL (5V) of *n*-heptane was then added as a poor solvent to slowly precipitate a solid. Stirring was carried out at 5-15 °C for 1 h, and 80 mL (16V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 50.5%. The resulting solid was subjected to XRPD and DSC analysis, and the results showed that the pyrazolopyrimidine ester compound was obtained in the crystalline form Form II.

### Example 13

2 g of the amorphous compound of the formula (I) with purity of 99% was weighed and dissolved at 0-5 °C in 10 mL (5V) of acetone as a good solvent. 6 mL (3V) of *n*-heptane was then added as a poor solvent to slowly precipitate a solid. Stirring was carried out at 0-5 °C for 1 h, and 24 mL (12V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 90.0%. The resulting solid was subjected to XRPD and DSC analysis, and the results showed that the pyrazolopyrimidine ester compound was obtained in the crystalline form Form II.

### Example 14

2 g of the amorphous compound of the formula (I) with purity of 97% was weighed and dissolved at 0-5 °C in 10 mL (5V) of acetone as a good solvent. 6 mL (3V) of n-heptane as a poor solvent and 0.002 g of crystal seeds in the crystalline form Form II were then added to slowly precipitate a solid. Stirring was carried out at 0-5 °C for 1 h, and 24 mL (12V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 89.3%. The resulting solid was subjected to XRPD and DSC analysis, and the results showed that the pyrazolopyrimidine ester compound was obtained in the crystalline form Form II.

### Example 15

2 g of the compound of the formula (I) in the crystalline form Form I was weighed and dissolved at 0-5 °C in 10 mL (5V) of acetone as a good solvent. 6 mL (3V) of *n*-heptane was then added as a poor solvent to slowly precipitate a solid. Stirring was carried out at 0-5 °C for 1 h, and 24 mL (12V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 92.2%. The resulting solid was subjected to XRPD and DSC analysis, and the results showed that the pyrazolopyrimidine ester compound was obtained in the crystalline form Form II.

### Example 16

2 g of the amorphous compound of the formula (I) with purity of 99% was weighed and dissolved at 0-5 °C in 10 mL (5V) of 2-methyltetrahydrofuran as a good solvent. 10 mL (5V) of *n*-heptane was then added as a poor solvent to slowly precipitate a solid. Stirring was carried out at 0-5 °C for 1 h, and 20 mL (10V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 94.0%. The resulting solid was subjected to XRPD and DSC analysis, and the results showed that the pyrazolopyrimidine ester compound was obtained in the crystalline form Form II.

### Example 17

2 g of the amorphous compound of the formula (I) with purity of 97% was weighed and dissolved at 0-5 °C in 10 mL (5V) of 2-methyltetrahydrofuran as a good solvent. 10 mL (5V) of *n*-heptane as a poor solvent and 0.002 g of crystal seeds in the crystalline form Form II were then added to slowly precipitate a solid. Stirring was carried out at 0-5 °C for 1 h, and 20 mL (10V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 93.4%. The resulting solid was subjected to XRPD and DSC analysis, and the results showed that the pyrazolopyrimidine ester compound was obtained in the crystalline form Form II.

### Example 18

2 g of the compound of the formula (I) in the crystalline form Form I was weighed and dissolved at 0-5 °C in 10 mL (5V) of 2-methyltetrahydrofuran as a good solvent. 10 mL (5V) of *n*-heptane was then added as a poor solvent to slowly precipitate a solid. Stirring was carried out at 0-5 °C for 1 h, and 20 mL (10V) of *n*-heptane was then slowly added dropwise within 30 min. After the addition was completed, stirring was continued at 0-5 °C for 1 h, followed by filtering. The resulting solid was dried under vacuum at 50 °C for 4.5 h, giving a solid at a purification yield of 94.8%. The resulting solid was subjected to XRPD and DSC analysis, and the results showed that the pyrazolopyrimidine ester compound was obtained in the crystalline form Form II.

### Example 19: Stability Study

Samples of the amorphous and crystalline (Forms I and II) compound of formula (I) were stored under different conditions. Changes in purity of them were then analyzed, and results are shown in the table below.

Storage conditions for the crystalline form Form II: at 60 °C for 10 days; at 60 °C and 75% RH (relative humidity) for 10 days; in light for 10 days; and at room temperature for 10 days.

Storage conditions for the amorphous and crystalline (Form I) forms: at 40 °C for 10 days; at 60 °C and 75% RH for 10 days; in light for 10 days; and at room temperature for 10 days.

Noticeable degradation of the amorphous sample that had been stored at 40 °C for 10 days and almost complete degradation of the amorphous sample that had been stored at 60°C and 75% RH for 10 days were observed. Form I samples showed lower degrees of degradation than the amorphous form, in particular when stored at room temperature and in light for 10 days, remaining stable. The Form II samples were more stable, in particular when stored at room temperature and in light, and were stable under degradation conditions such as high-temperature and high-humidity. The stability Form II of was obviously better than the amorphous form and even better than Form I.

The crystalline form Form II has demonstrated its value in terms of stability - it allows long-term storage without special requirements in respect of temperature, light, humidity or oxygen level.

| | Initial (Day 0) | 40 °C (Day 10) | 60 °C (Day 10) | 60 °C + 75% RH (Day 10) | In Light (Day 10) | Room Temperature (Day 10) |
|---|---|---|---|---|---|---|
| Amorphous | 99.07% | 96.10% | - | 0.27% | 98.63% | 98.01% |
| Form I | 98.68% | 98.60% | - | 90.62% | 98.62% | 98.64% |
| Form II | 99.13% | - | 98.90% | 97.80% | 99.17% | 99.13% |

## Claims

1. A crystalline form Form II of a pyrazolopyrimidine ester compound of the structure of formula (I), **characterized by** 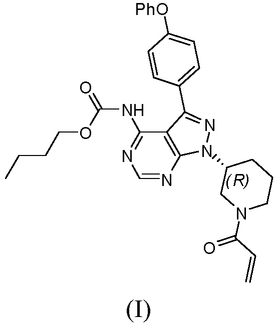 having characteristic peaks expressed in degrees 2θ at 5.53°, 9.82°, 10.76°, 17.87°, 20.55°, 21.28°, 21.92° and 25.15° in its X-ray powder diffraction (XRPD) pattern, wherein the error range of degrees 2θ is ± 0.20°, and the XRPD pattern is obtained using Cu-Kα radiation.

2. The crystalline form Form II of the pyrazolopyrimidine ester compound according to claim 1, **characterized by** having characteristic peaks expressed in degrees 2θ at 5.53°, 9.82°, 10.76°, 14.25°, 14.81°, 16.72°, 17.87°, 18.33°, 19.06°, 19.67°, 20.55°, 20.78°, 21.28°, 21.92°, 22.93°, 23.38°, 23.85°, 25.15°, 26.09°, 26.51°, 26.97°, 27.52°, 27.90°, 28.37°, 29.00°, 29.70°, 31.57°, 32.57°, 33.94° and 36.71° in the XRPD pattern, wherein the error range of degrees 2θ is ± 0.20°.

3. The crystalline form Form II of the pyrazolopyrimidine ester compound according to claim 2, **characterized by** the XRPD pattern as shown in Fig. 1.

4. The crystalline form Form II of the pyrazolopyrimidine ester compound according to claim 1, **characterized by** being an irregularly shaped crystalline form and having an endothermic peak at 129.59 °C in its differential scanning calorimetry (DSC) curve, wherein the error range of the endothermic peak temperature is ± 1.00 °C.

5. The crystalline form Form II of the pyrazolopyrimidine ester compound according to claim 4, **characterized by** the DSC curve as shown in Fig. 2.

6. The crystalline form Form II of the pyrazolopyrimidine ester compound according to claim 1, **characterized by** having characteristic absorption bands at wavenumbers of 1030, 1078, 1138, 1197, 1233, 1277, 1376, 1445, 1455, 1488, 1504, 1556, 1588, 1606, 1642, 1750, 2865, 2897, 2928, 2954, 3012, 3027, 3044, 3076, 3166 and 3195 cm⁻¹ in its infrared (IR) spectrum, wherein the error range of the absorption band peaks is ± 2 cm⁻¹.

7. The crystalline form Form II of the pyrazolopyrimidine ester compound according to claim 6, **characterized by** the IR spectrum as shown in Fig. 3.

8. A method of preparing the crystalline form Form II of the pyrazolopyrimidine ester compound according to any of claims 1 to 7, **characterized in** comprising the steps of:
dissolving the pyrazolopyrimidine ester compound of the structure of formula (I) in a good solvent; then adding a poor solvent to precipitate a solid; and stirring, followed by filtering and drying, affording the pyrazolopyrimidine ester compound in the crystalline form Form II,
wherein the pyrazolopyrimidine ester compound of the structure of formula (I) is amorphous or in a crystalline form Form I, the crystalline form Form I having characteristic peaks expressed in degrees 2θ at 11.26°, 14.03°, 14.80°, 17.07°, 19.78°, 21.21°, 22.39° and 23.85° in its XRPD pattern;
wherein the good solvent is selected from acetone, 2-methyltetrahydrofuran, methanol, acetonitrile, ethyl acetate and tetrahydrofuran, and the poor solvent is selected from *n*-heptane, methyl *tert*-butyl ether, water and cyclohexane; and the good solvent is used at a volume-to-weight ratio of 5-10 to the compound of formula (I), and the poor solvent is used at a volume-to-weight ratio of 10-150 to the compound of formula (I).

9. The method of preparing the crystalline form Form II of the pyrazolopyrimidine ester compound according to claim 8, **characterized in that** the poor solvent is added slowly in batches.

10. Use of the crystalline form Form II of the pyrazolopyrimidine ester compound according to any of claims 1 to 7, **characterized in that** it is used to prepare a medicament for treating lymphoma and lymphocytic leukemia.
